# EUROPEAN PATENT APPLICATION

(11) **EP 1 471 140 A1**
(43) Date of publication of application: **27.10.2004**
(21) Application number: 03734902.4
(22) Date of filing: 31.01.2003
(51) Int. Cl.: C12N 5/02

(54) **LIQUID FOR FROZEN STORAGE OF PRIMATE EMBRYO STEM CELLS AND FROZEN STORAGE METHOD**

(30) Priority: 31.01.2002 JP 2002024382
(71) Applicant: ASAHI TECHNO GLASS COSPORATION, Chiba 273-0044 (JP)
(72) Inventor: NAKATSUJI, Norio, Kyoto-shi, Kyoto 606-0014 (JP); SUEMORI, Hirofumi Pearl Haitsu Inari 3001, Kyoto 612-0012 (JP); ASAKA, Isao, Asahi Techno Glass Corporation, Chiba 273-0044 (JP)
(74) Representative: Müller-Boré & Partner Patentanwälte
(86) International application number: PCT/JP2003/000999
(87) International publication number: WO 2003/064634

(57) **Abstract**

A cryopreservation medium and a cryopreservation method which make it possible to cryopreserve ES cells from primates simply with high viability are provided.

A cryopreservation medium containing a cryoprotectant at a concentration of from 12% (W/V) to 50% (W/V) and a cryopreservation method for primate embryonic stem cells, which comprises a step of suspending primate embryonic stem cells in the cryopreservation medium, and a refrigeration step of freezing the suspension of the primate embryonic stem cells in the cryopreservation medium by cooling it to -80°C or below at a rate of from 0.5°C to 10°C per minute, and a preservation step of storing the frozen suspension of primate embryonic stem cells in the cryopreservation medium enable simple cryopreservation of primate embryonic stem cells with high viability.

## Description

### TECHNICAL FIELD

The present invention relates to a cryopreservation medium and a cryopreservation method for embryonic stem (ES) cells from primates including humans, which have the potential to differentiate into any tissues in the body and promising applications in the fields of cell culture, tissue transplantation, research for drug discovery and gene therapy.

### BACKGROUND ART

Embryonic stem cells are cell lines derived from the inner cell mass of the blastocyst. When undifferentiated, they are pluripotent and can contribute to formation of any tissues including the germ line. Experiments using animals such as mice showed that blastocysts or morulae having ES cells injected therein or morulae with clumps of ES cells attached (Wood, S.A. et al., Proc. Natl. Acad. Sci. USA 90:4582-4585 (1993)) develop into progeny having two different genomes (which is called chimeric progeny). Moreover, ES cells are used in numerous medical studies to develop animal models of human diseases (Smithies, O. et al., Proc. Natl. Acad. Sci. USA:5266-5272 (1995)) and cell differentiation study models (Doetschman, T.C. et al., J. Embryol. Exp. Morph. 87:27-45 (1985)).

On the other hand, the report of successful cloning by transplantation of adult somatic nuclei by Wilmu et al. in 1997 (Wilmut I. et al., Nature 385:810-813 (1997)) implies the possibility of formation of genetically identical embryos. Furthermore, recently reported techniques for inducing differentiation of ES cells into various tissues including the nervous tissue (Svendsen C. N. et al., TINDS 22:357-364 (1999), Li M. et al., Curr. Biol. 8:971-974 (1998), Okabe S. et al., Mech. Dev. 59:89-102 (1996)) have raised expectation of medical transplantation of genetically identical tissues without immune elimination and gene therapies using ES cells derived from cloned embryos.

Under the above-mentioned circumstances, the target for ES cell production extended from small laboratory animals to primates including humans, and Thomson et al. reported isolation of rhesus monkey ES cells in 1995 (1995 Proc. Natl. Acad. Sci. USA 92:7844-7848; USP5,843,780; USP6,200,806 and WO96/22362). Further, in 1998, isolation of ES cells similarly derived from in vitro fertilized human ova was reported (1998 Science Nov 6;282(5391):1145-7).

Suemori et al. succeeded in establishment of ES cell lines from intracytoplasmic sperm injected ova of cynomolgus monkeys, which are used widely in medical studies for their usefulness in preclinical testing, and demonstrated that they retain their pluripotency for a long term (2001 Dev. Dyn. Oct; 222(2):273-9; and JP-A-2002-159289). The potency of the cynomolgus monkey ES cells to differentiate to dopaminergic neurons was also confirmed (2002 Proc. Natl. Acad. Sci. USA 99:1580-1585).

However, the ES cells of primates including humans established by the above method have a problem of poor cryopreservation efficiency under the conventional cryopreservation conditions mainly used for mouse ES cells. Therefore, when frozen according to conventional protocols, they take at least 2 to 4 weeks to proliferate in culture enough to be subcultured.

Besides the above-mentioned cryopreservation protocols for mouse ES cells, among reports on stem cell cryopreservation, a cryopreservation composition for nucleated cells including hematopoietic stem cells (JP-A-2000-201672) has already been reported. However, a method applicable to primate ES cells has not been reported yet.

The object of the present invention is to provide a cryopreservation medium and a cryopreservation method which make it possible to cryopreserve ES cells from primates including humans, if not known yet, simply with high viability.

### DISCLOSURE OF THE INVENTION

The present inventors have carried out studies to solve the above-mentioned problems and have completed the present invention on the basis of the finding that the use of a cryopreservation medium containing a cryoprotectant at a higher concentration than ever, specifically at a concentration of from 12% (W/V) to 50% (W/V), allows cryopreservation of primate ES cells with easy control at low cost.

Namely, the present invention provides the following invention.
1. A cryopreservation medium for primate embryonic stem cells containing a cryoprotectant at a concentration of from 12% (W/V) to 50% (W/V).
2. The cryopreservation medium according to 1, wherein the concentration of the cryoprotectant is from 15% (W/V) to 30% (W/V).
3. The cryopreservation medium according to 1 or 2, which contains at least one component selected from the group consisting of dimethyl sulfoxide, glycerin, ethylene glycol, propylene glycol and polyvinyl pyrrolidone as the cryoprotectant.
4. The cryopreservation medium according to any one of 1 to 3, wherein the cryoprotectant is dimethyl sulfoxide and/or glycerin.
5. The cryopreservation medium according to 4, wherein the cryoprotectant is dimethyl sulfoxide, and its concentration is from 15% (W/V) to 30% (W/V).
6. The cryopreservation medium according to 4, wherein the cryoprotectant is glycerin, and its concentration is from 12% (W/V) to 30% (W/V).
7. The cryopreservation medium according to any one of 1 to 6, which contains, in addition to the cryoprotectant, serum and/or a serum replacement at a concentration of from 10% (W/V) to 85% (W/V).
8. The cryopreservation medium according to any one of 1 to 6, which contains, in addition to the cryoprotectant, serum at a concentration of from 10% (W/V) to 50% (W/V).
9. The cryopreservation medium according to 7 or 8, wherein the serum is fatal bovine serum.
10. The cryopreservation medium according to 7, wherein the serum replacement contains at least one component selected from the group consisting of albumin or an albumin substitutes, transferrin or transferrin substitutes, and insulin or insulin substitutes.
11. The cryopreservation medium according to 7 or 10, wherein the serum replacement contains at least one component selected from the group consisting of albumin or albumin substitutes, transferrin or transferrin substitutes, and insulin or insulin substitutes and at least one component selected from the group consisting of amino acids, vitamins, antioxidants, collagen precursors and trace elements.
12. The cryopreservation medium according to any one of 7 to 11, which contains, in addition to the cryoprotectant and serum and/or serum replacement, a basal medium for animal tissue culture containing at least from 0.3% (W/V) to 5% (W/V) of glucose, at a concentration of from 10% (W/V) to 75% (W/V).
13. A cryopreservation method for primate embryonic stem cells using the cryopreservation medium as defined in any one of 1 to 12, which comprises a step of suspending primate embryonic stem cells in the cryopreservation medium, and a refrigeration step of freezing the suspension of the primate embryonic stem cells in the cryopreservation medium by cooling it to -80°C or below, and a preservation step of storing the frozen suspension of primate embryonic stem cells in the cryopreservation medium.
14. The cryopreservation method according to 13, wherein the cooling rate in the refrigeration step of freezing the suspension of the primate embryonic stem cells in the cryopreservation medium by cooling it to -80°C or below is from 0.5°C to 10°C per minute.

### BRIEF DESCRIPTION OF THE DRAWINGS

Fig. 1 shows the time course of the alkaline phosphatase activity of post-thaw cynomolgus monkey ES cells in culture in Example 1. % denotes % (V/V).
Fig. 2 is a photograph of ES cell colonies after a post-thaw passage in Example 1 (magnification 40).
Fig. 3 is a photograph of ES cell colonies after a post-thaw passage in Example 1 (magnification 100).
Fig. 4 is a photograph of ES cell colonies after a post-thaw passage stained with an alkaline phosphatase substrate in Example 1.
Fig. 5 shows the time course of the alkaline phosphatase activity of post-thaw cynomolgus monkey ES cells in culture in Example 1. % denotes % (V/V).
Fig. 6 is a photograph of ES cell colonies 5 days after thawing in Example 2 (magnification 40).
Fig 7 is a photograph of ES cell colonies 5 days after thawing in Example 2 (magnification 100).
Fig 8 is a photograph of ES cell colonies 7 days after thawing in Example 2 (magnification 40).
Fig 9 is a photograph of ES cell colonies 7 days after thawing in Example 2 (magnification 100).
Fig 10 is a photograph of ES cell colonies 7 days after thawing stained with an alkaline phosphatase substrate in Example 2.
Fig. 11 is a photograph of feeder cells in culture stained with an alkaline phosphatase substrate in Example 2.
Fig. 12 is a photograph of ES cell colonies 5 days after thawing in the comparative experiment in Example 2 (magnification 40).
Fig. 13 is a photograph of ES cell colonies 5 days after thawing in the comparative experiment in Example 2 (magnification 100).
Fig. 14 is a photograph of ES cell colonies 7 days after thawing in the comparative experiment in Example 2 (magnification 40).
Fig. 15 is a photograph of ES cell colonies 7 days after thawing in the comparative experiment in Example 2 (magnification 100).
Fig. 16 is a photograph of ES cell colonies 7 days after thawing stained with an alkaline phosphatase substrate in the comparative experiment in Example 2.
Fig. 17 is a HE-stained section of a teratoma formed in Example 3.
Fig. 18 is a graph showing the time course of ALP activity at various DMSO concentrations in Example 4.
Fig. 19 is a graph showing the correlation between DMSO concentration and cell count 7 days after thawing in Example 4. % denotes % (V/V).
Fig. 20 is a graph showing the time course of the ALP activity of post-thaw cynomolgus monkey ES cells in culture after cryopreservation in the presence of glycerin in Example. % denotes % (V/V).
Fig. 21 is a graph showing the cell count in the 8day culture of the recovered ES cells after cryopreservation in the presence of glycerin in Example 5. % denotes % (V/V).
Fig. 22 is a graph showing the number of colonies in the 8 day culture of the recovered ES cells after cryopreservation in the presence of glycerin in Example 5. % denotes % (V/V).
Fig. 23 is a graph showing the time course of ALP activity of post-thaw ES cells in culture after cryopreservation at different storage cell densities in Example 6. % denotes % (V/V).
Fig. 24 is a graph showing the cell count in the 8 day culture after cryopreservation at different cell densities in Example 6. % denotes % (V/V).
Fig. 25 is a graph showing the time course of ALP activity of post-thaw ES cells in culture after cryopreservation in cryopreservation media supplemented with serum or a serum replacement in Example 7. % denotes % (V/V).
Fig. 26 is a graph showing the cell count in the 7 day culture after cryopreservation in cryopreservation media supplemented with serum or a serum replacement in Example 7. % denotes % (V/V).
Fig. 27 is a graph showing the time course of ALP activity of recovered ES cells in culture after cryopreservation in the presence of 20% of a serum replacement in Example 8. % denotes % (V/V).
Fig. 28 is a graph showing the cell count in the 7 day culture after cryopreservation in the presence of 20% of a serum replacement in Example 8. % denotes % (V/V).

### BEST MODE FOR CARRYING OUT THE INVENTION

The cryopreservation medium useful for cryopreservation of primate embryonic stem cells of the present invention preferably contains at least one member selected from dimethyl sulfoxide (hereinafter referred to simply as DMSO), glycerin, ethylene glycol, propylene glycol and polyvinyl pyrrolidone as a cryoprotectant. These may be contained singly or in combination. The particularly suitable cryoprotectant is DMSO because of its permeability to ES cells, and the second suitable cryoprotectant is glycerin.

The concentration of the cryoprotectant which ensures the effects of the present invention is from 12% (W/V) to 50% (W/V), suitably from 15% (W/V) to 30% (W/V). The values are selected by the balance between cryoprotection effects and toxicity, and their approximations are also covered by the present invention as far as they meet the object of the present invention. The cryoprotection effects are weak at too low a concentration, while because of the reports that embryonic stem cells and embryonic carcinoma cells in culture begin to differentiate in 4 days at a DMSO concentration of above 1% (W/V) (Nicholas et al., Development (1994) 120: 1473-1482, Steven, Development (1994) 120: 3301-3312) and the possibility of carryover contamination of cell cultures, it is not preferred to use the cryoprotectant at an excessively high concentration.

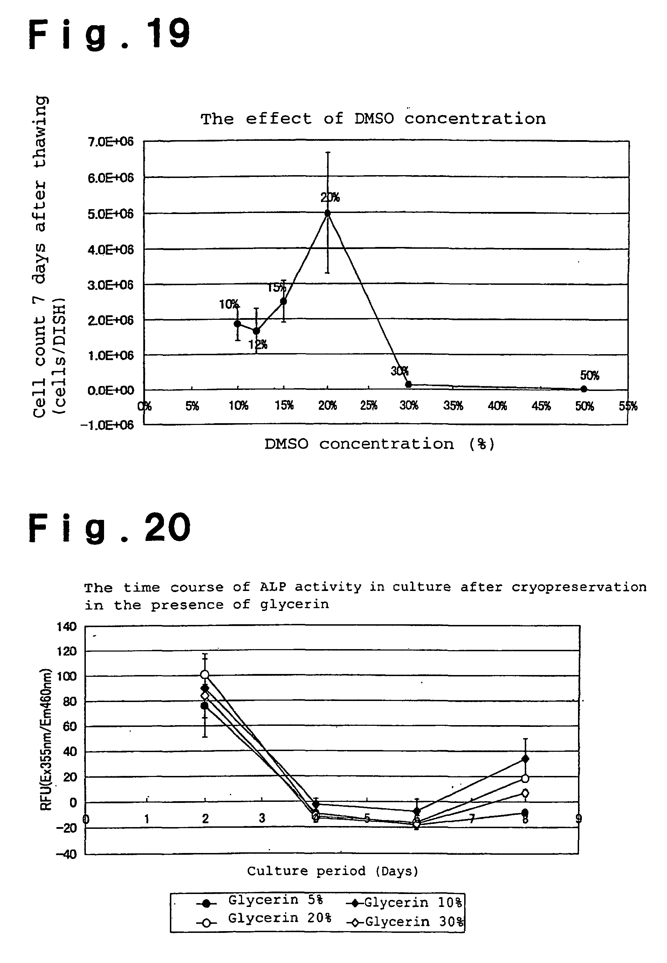

When the cryoprotectant is DMSO, the concentration in the cryopreservation medium is suitably from 15% (W/V) to 30% (W/V), particularly suitably from 15% (W/V) to 25% (W/V) (Fig. 18 and Fig. 19). When the cryoprotectant is glycerin, the concentration in the cryopreservation medium is particularly suitably from 12% (W/V) to 30% (W/V) (Figs. 20 to 22).

A previous report on a cryopreservation composition for nucleated cells (JP-A-2000-201672) discloses a cryopreservation composition containing from 3% (W/V) to 20% (W/V) of DMSO in combination with dextran and albumin. However, it merely refers to the conventionally used maximum and minimum concentrations ("Soshiki Baiyo no Gijutsu" edited by the Japanese Tissue Culture Association (1982) p.42-44, "Shin-Seikagaku Jikken Koza vol. 18 "Saibo no Baiyo Gijutsu" edited by the Japanese Biochemical Society (1990) p.14-15) and does not suggest that the single use of DMSO is especially effective in cryopreservation of nucleated cells. It is difficult to expect by analogy that within this concentration range, the present invention has specific effects on primate embryonic stem cells.

In addition, DMSO is considered to be harmful at concentrations of 10% or above ("Bunshi-Saibo-Seibutsugaku Jiten" edited by Muramatsu Masami et al. (1997) p.391) so far, and experiments using chicken lung cells did not give high viabilities at DMSO or glycerin concentrations as high as from 15 to 20% (Dougherty, R, M. Nature 193:550(1962)). Therefore, successful cryopreservation of primate embryonic stem cells at high cryoprotectant concentrations as in the present invention is a totally new finding.

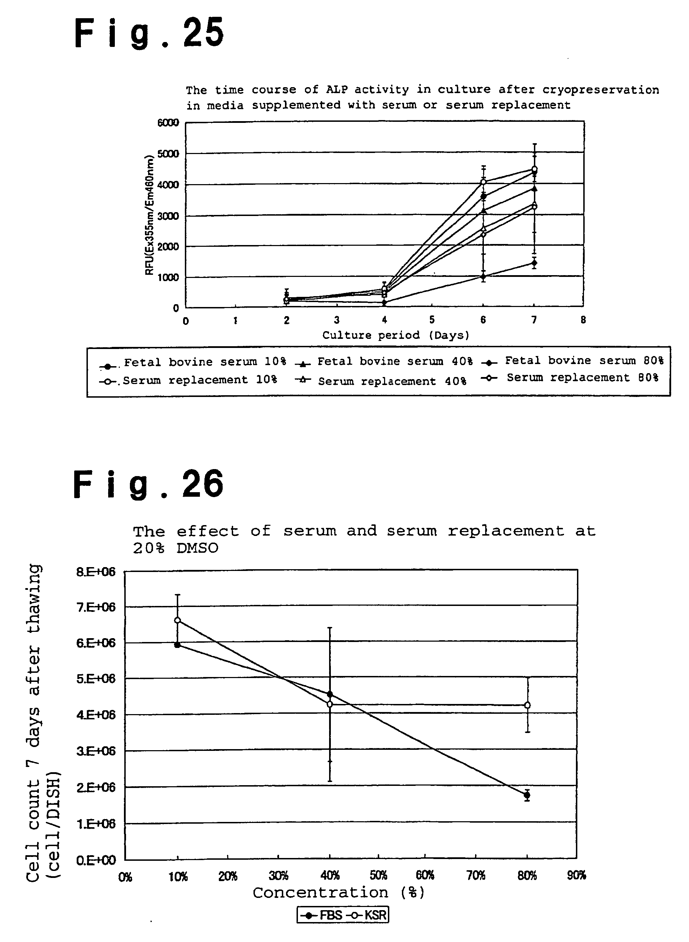

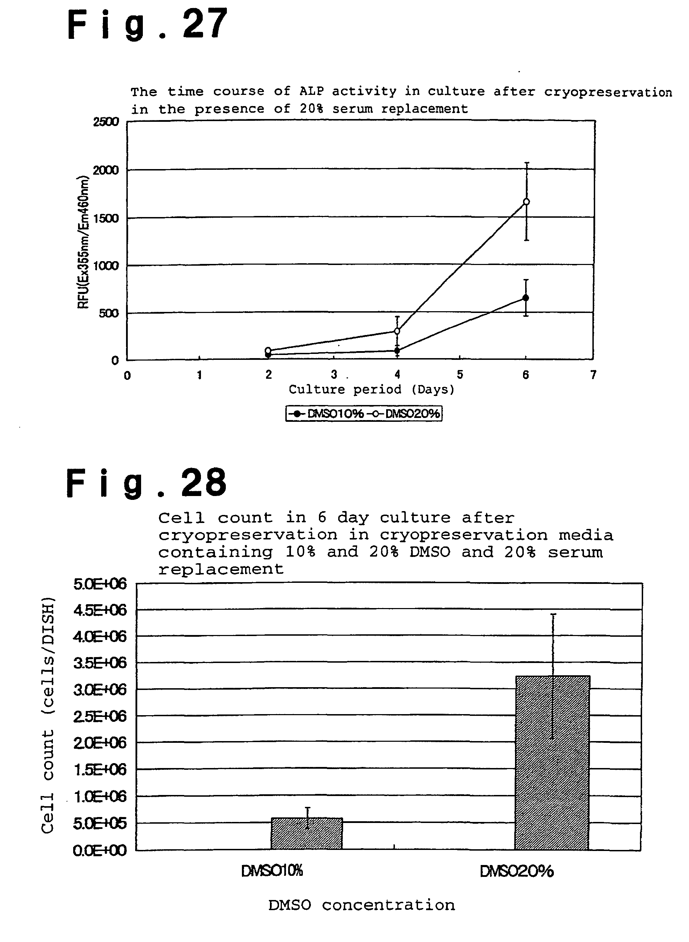

The cryopreservation medium of the present invention suitably contains serum and/or a serum replacement at a concentration of from 10% (W/V) to 85% (W/V). As the serum, FBS is suitably used. The serum replacement suitably contains at least one component selected from the group consisting of albumin or albumin substitutes, transferrin or transferrin substitutes, and insulin or insulin substitutes. Particularly suitably, a serum replacement is used to maintain embryonic stem cells in an undifferentiated stage. It seems that serum and/or a serum replacement is essential at least at the cryopreservation. Excluding the cryoprotectant, serum or a serum replacement may constitute the rest of the cryopreservation medium of the present invention. However, the cryopreservation medium is preferred to contain serum and/or a serum replacement at a low concentration (Figs. 25 and 26). A final concentration of at least 10% (W/V) seems necessary to ensure cell stabilization. The particularly suitable concentration of serum and/or a serum replacement is from 10% (W/V) to 50% (W/V).

A serum replacement containing at least albumin or albumin substitutes is suitable. In particular, a serum replacement containing at least two components, albumin or albumin substitutes and transferrin or transferrin substitutes, is particularly suitable. The optimum serum replacement contains at least three components, albumin or albumin substitutes, transferrin or transferrin substitutes, and insulin or insulin substitutes.

It is preferred that the serum replacement further contains at least one component selected from the group consisting of amino acids, vitamins, antioxidants, collagen precursors and trace elements. Serum may be supplemented with these components, if necessary.

In the present invention, the albumin is suitably bovine serum albumin (BSA), fetal bovine albumin (fetuin), human serum albumin (HAS), a recombinant human albumin (rHSA), ovalbumin or the like. The albumin substitutes are suitably bovine pituitary extract, bovine embryo extract, chicken extract, vegetable albumin or the like.

The transferrin is suitably iron-bound transferrin or iron-free transferrin. In the latter case, coexistence of iron ions is suitable. The transferrin substitutes are suitably a soluble iron compound or an iron ion. For example, iron chelate compounds such as the ferric citrate chelate or the ferrous sulfate chelate is preferred. As the insulin, an insulin bound to a metal ion such as the zinc ion or metal-free insulin is suitable. In the latter case, coexistence of a metal ion is suitable. As the insulin substitutes, a soluble metal compound or a metal ion is suitable. As the metal, zinc is suitable. Zinc chloride, zinc bromide or zinc sulfide heptahydrate may specifically be mentioned.

In the present invention, as the amino acids, glycine, L-alanine, L-asparagine, L-cysteine, L-asparaginic acid, L-glutaminic acid, L-phenylalanine, L-histidine, L-isoleusine, L-lysine, L-leucine, L-glutamine, L-arginine, L-methionine, L-proline, L-hydroxyproline and L-valine may be mentioned. As the vitamins, vitamins in the vitamin B group such as biotin, pantothenic acid, choline, folacin (folic acid), myoinositol, niacin, pyridoxine, riboflavin, thiamine and cobalamin are suitable. As the antioxidants, glutathione and ascorbic acid may be mentioned. As the collagen precursors, a poly(L-proline) or its derivatives, or a poly(L-hydroxyproline) or its derivatives may be mentioned. As the trace elements, in addition to previously mentioned iron and zinc, Ag⁺, Al³⁺, Ba²⁺, Cd²⁺, Co²⁺, Cr³⁺, Ge⁴⁺, Mn²⁺, Mo⁶⁺, Ni²⁺, Rb⁺, Se⁴⁺, Si⁴⁺, Sn²⁺, V⁵⁺, Zr⁴⁺, Br⁻, F⁻, I⁻ and the like may be mentioned.

The above-mentioned components such as amino acids, vitamins, antioxidants, collagen precursors and trace elements may be used in combinations, of two or more of the same type or different types.

The cryopreservation medium of the present invention may further contain a basal medium for animal tissue culture at a concentration of from 10% (W/V) to 75% (W/V). As the basal medium, a basal medium for animal tissue culture containing at least from 0.3% (W/V) to 5% (W/V) of glucose is suitably used at a concentration of from 10% (W/V) to 75% (W/V) because of recovery of thawed cells and suppression of apoptosis. The selected glucose content reflects the fact that though animal tissue culture media are usually used at a minimum glucose concentration of about 0.1%, media with slightly higher glucose content are usually used for embryonic stem cells. In addition to the cryoprotectant and serum and/or the serum replacement, a basal medium suitable for culture of the cells is incorporated in the cryopreservation medium. A basal medium is added so that the concentration of the cryoprotectant and serum and/or the serum replacement is the essential minimum (about 25% (W/V) in total), while the basal medium constitutes the other 75% (W/V). When addition of a basal medium to the cryopreservation medium is essential, the minimum amount of it necessary to obtain its effect is 10% (W/V).

When primate embryonic stem cells are cryopreserved according to the present invention, the above-mentioned cryoprotectant is used to cryopreserve harvested primate embryonic stem cells, namely, by a method comprising a step of suspending primate embryonic stem cells in a cryopreservation medium containing the cryoprotectant at a concentration of from 12% (W/V) to 50% (W/V), a refrigeration step of freezing the suspension of the primate embryonic stem cells in the cryopreservation medium by cooling it to -80°C or below, and a preservation step of storing the frozen suspension of primate embryonic stem cells in the cryopreservation medium. In the refrigeration step, the cooling rate is preferably from 0.5°C to 10°C per 1 minute, because rapid cooling leads to difference in ice formation between the intracellular water and the extracellular water which is destructive of the cellular microstructure. The preservation step can be performed at -80°C or below preferably in liquid nitrogen and/or liquid nitrogen vapor for more stable storage.

The details of the mechanism underlying the present invention are not clear. Though primate embryonic stem cells are supposed to be more susceptible to cryodamage than mouse embryonic stem cells, it is speculated that the use of a cryoprotectant at higher concentrations than usual suppresses cryodamage and remarkably improves cell viability. The present invention provides a cryopreservation medium and a cryopreservation method which allow simple cryopreservation of primate embryonic stem cells with high viability.

### EXAMPLES

Now the present invention will be described by referring to Examples and Comparative Examples. However, the Examples are mere embodiments of the present invention which help reproduction of the present invention and by no means limits or restricts the present invention. The specific gravities of the DMSO, glycerin, the serum replacement and the culture medium used were 1.1, 1.26, 1.0 and 1.0, respectively.

### EXAMPLE 1

Cynomolgus monkey ES cells were subcultured for 33 passages and dissociated into cell suspension from three 90-mm Petri dishes, and the cell suspension was centrifuged at 1,000 rpm for 5 minutes. The supernatant was removed to obtain a cell precipitate. The cell precipitate was resuspended in 1 ml of a cryopreservation medium containing 20% (V/V) [= 22% (W/V)] of DMSO, 40% (V/V) of a serum replacement containing albumin, insulin and transferrin (Knockout Serum Replacement: Invitrogen) [hereinafter referred to as serum replacement (A)] and 40% (V/V) of DMEM:Ham F12 = 1:1 medium [hereinafter referred to as medium (A)] supplemented with 3 g/l of glucose, 0.5 g/l of CaCl₂ 0.15 g/l of MgSO₄. The cell suspension was transferred to a serum tube, refrigerated to -80°C at a rate of about 1°C/min in Mr. Frosty (Nalgen) and then stored in a liquid nitrogen container for about 6 days.

In a comparative experiment, a cell suspension in 1 ml of a cryopreservation medium consisting of 0% (V/V) of DMSO, 50% (V/V) of serum replacement (A) and 50% (V/V) of medium (A) was prepared, transferred to a serum tube, refrigerated to -80°C at a rate of about 1°C/min in Mr. Frosty (Nalgen) and then stored in a liquid nitrogen container for about 6 days.

The cells were thawed, plated in 60-mm Petri dishes in duplicate and cultured in the medium described in Suemori et al. (2001 Dev. Dyn. Oct; 222(2):273-9).

ES cell proliferation was investigated by following the time course of the activity of alkaline phosphatase, which is a marker of ES cells. After 3, 5 and 7 days, the medium was removed from the dishes by aspiration, and the cells were washed with 1 ml of 30 mM Hepes buffer containing 0.1 mg/l of CaCl₂ and 0.1 mg/l of MgSO₄(7H₂O) and treated with 1 ml of 0.2 mM 4-methylumbelliferyl phosphate solution in the same buffer in an incubator at 37°C for 1 hour. 0.8 ml portions of the reaction mixture were transferred to a 24-well microplate and analyzed by fluorometry at Ex355/Em460. Fluorescence per 1 ml was calculated and plotted. As a control, the alkaline phosphatase activity of the same feeder cells as used during subcultures of ES cells was measured.

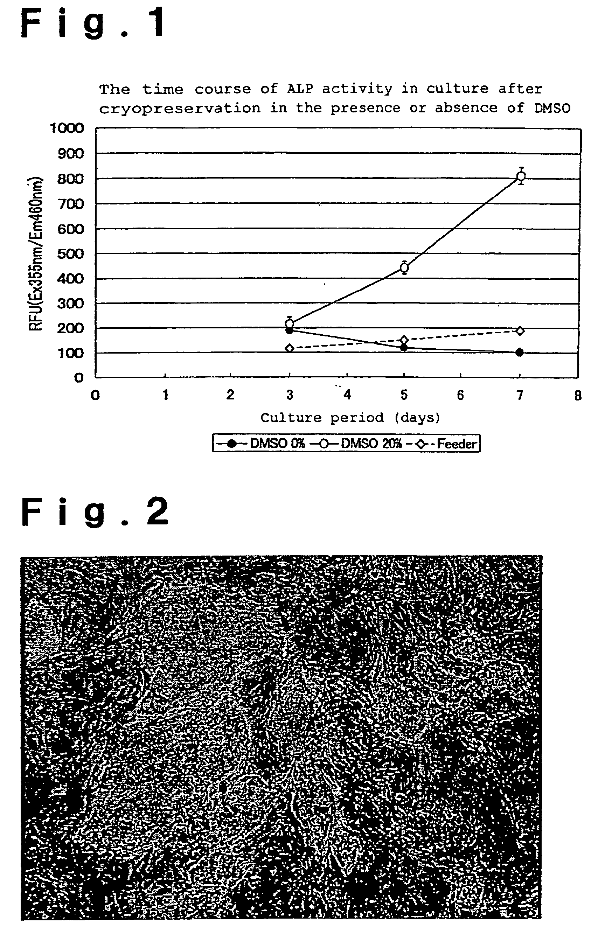

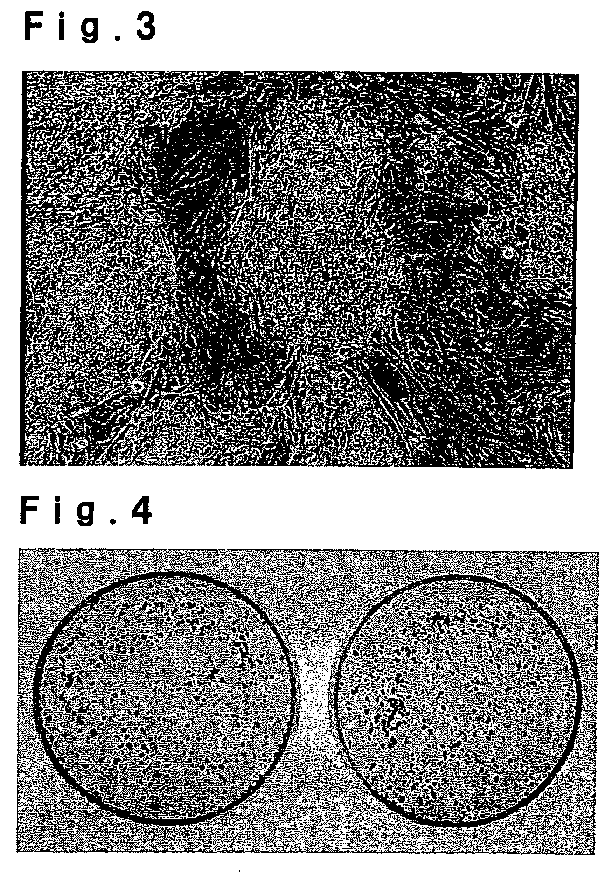

The cells in the illustrative experiment showed significantly higher alkaline phosphatase activity than the feeder cells (Fig. 1) and could be subcultured after 1 week. After three-fold dilution followed by 3 days of subculture, successful proliferation of ES cells was observed without formation of differentiated colonies (Figs. 2 and 3). After completion of the culture, the cells in each Petri dish were fixed with paraformaldehyde and ethanol and stained with Vector Red alkaline phosphatase substrate (Vector Labs). The numerous successfully stained colonies (Fig. 4) demonstrate that the ES cells cryopreserved according to the present invention remained normal.

In contrast, in the comparative experiment, the alkaline phosphatase activity was much the same as that in the feeder cells (Fig. 1), and no ES cell colonies were formed.

### EXAMPLE 2

Cynomolgus monkey ES cells subcultured for 34 passages were harvested from six 90-mm Petri dishes and treated by a method similar to that employed in Example 1 to give a cell precipitate. The cell precipitate was resuspended in 2 ml of a cryopreservation medium consisting of 20% (V/V) [= 22% (W/V)] of DMSO, 40% (V/V) of serum replacement (A), 40% (V/V) of medium (A). The cell suspension was transferred to two serum tubes, refrigerated to -80°C at a rate of about 1°C/min in Mr. Frosty (Nalgen) and stored in a liquid nitrogen container for 1 day.

In a comparative experiment, a cell suspension in 2 ml of a cryopreservation medium consisting of 10% (V/V) of DMSO, 45% (V/V) of serum replacement (A) and 45% (V/V) of medium (A) was prepared, transferred to two serum tubes, refrigerated to -80°C at a rate of about 1°C/min in Mr. Frosty (Nalgen) and then stored in a liquid nitrogen container for 1 day.

The cells in each serum tube were thawed, plated in 60-mm Petri dishes in duplicate and cultured in the medium described in Suemori et al. (2001 Dev. Dyn. Oct;222(2):273-9).

After 2, 4, 6 and 7 days, ES cell proliferation was assessed by alkaline phosphatase activity as described in Example 1.

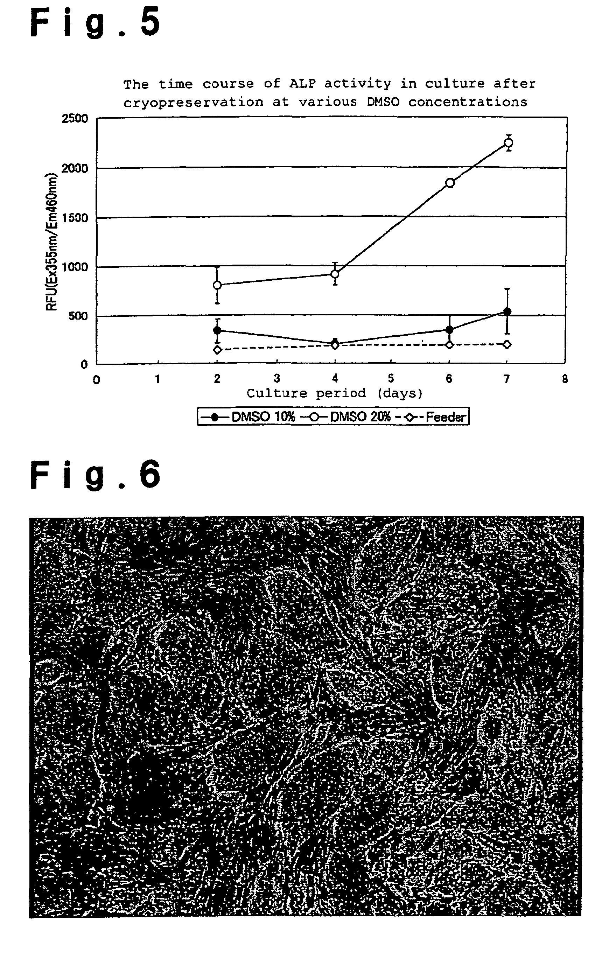

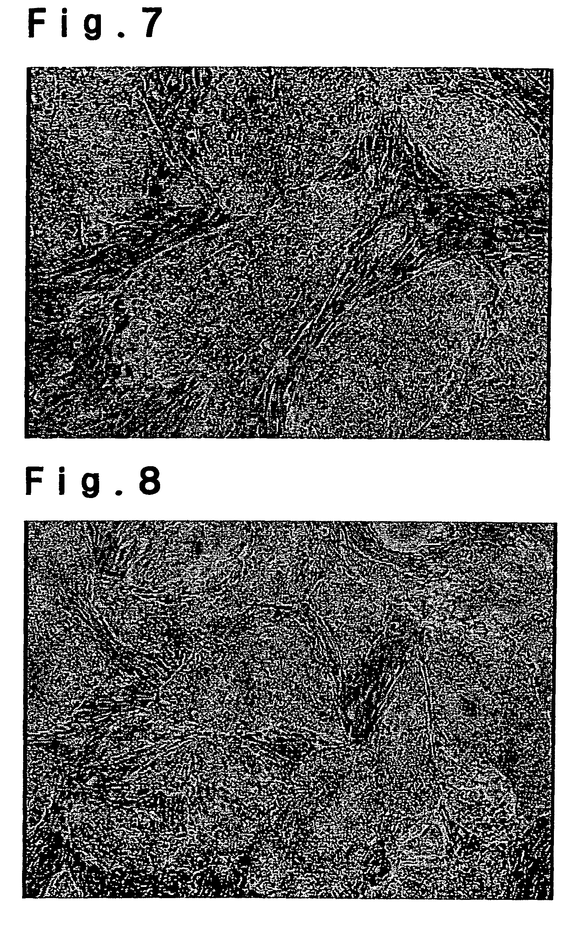

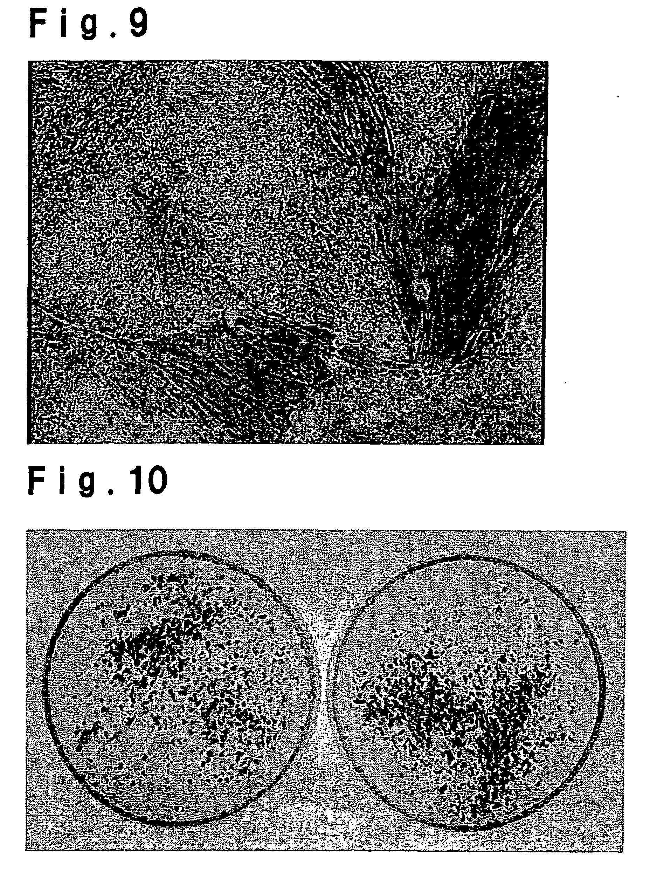

The cells in the illustrative experiment showed significantly higher alkaline phosphatase activity than the feeder cells (Fig. 5) and the cultures reached the stage at which they were able to be subcultured after 5 days (Figs. 6 and 7). After 7 days of prolonged culture, successful proliferation of ES cells was observed without formation of differentiated colonies (Figs. 8 and 9).

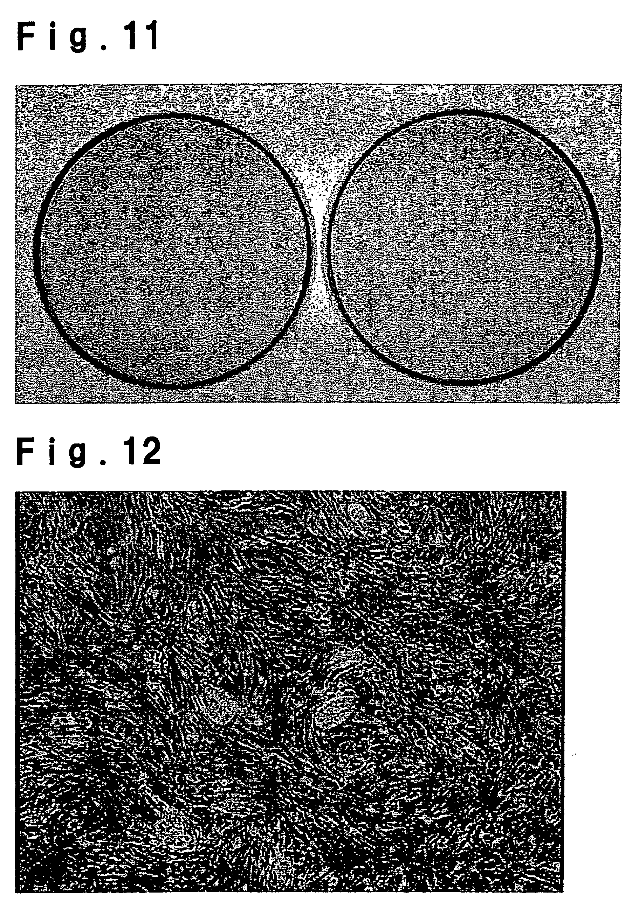

After completion of the culture, the cells in each Petri dish were fixed with paraformaldehyde and ethanol and stained with Vector Red alkaline phosphatase substrate (Vector Labs). The numerous successfully stained colonies (Fig. 10) demonstrate that the ES cells cryopreserved according to the present invention remained normal. Similar staining of the feeder cells gave no stained colonies (Fig. 11).

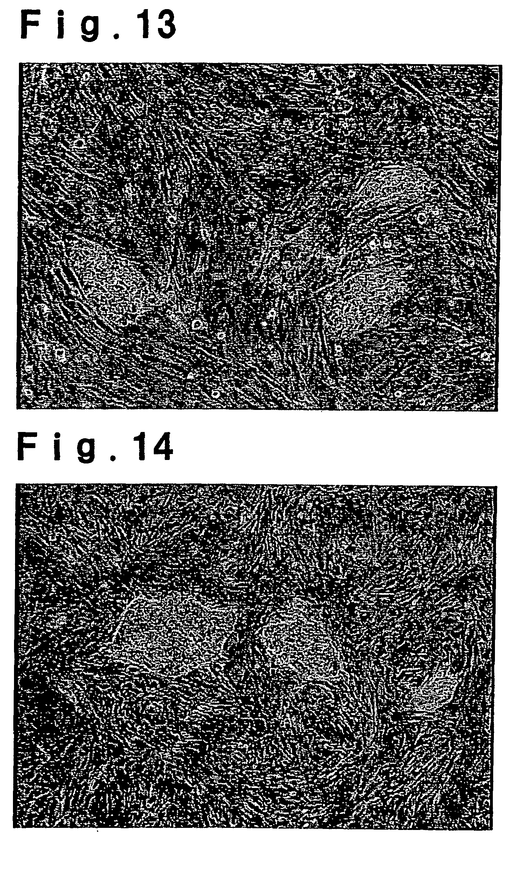

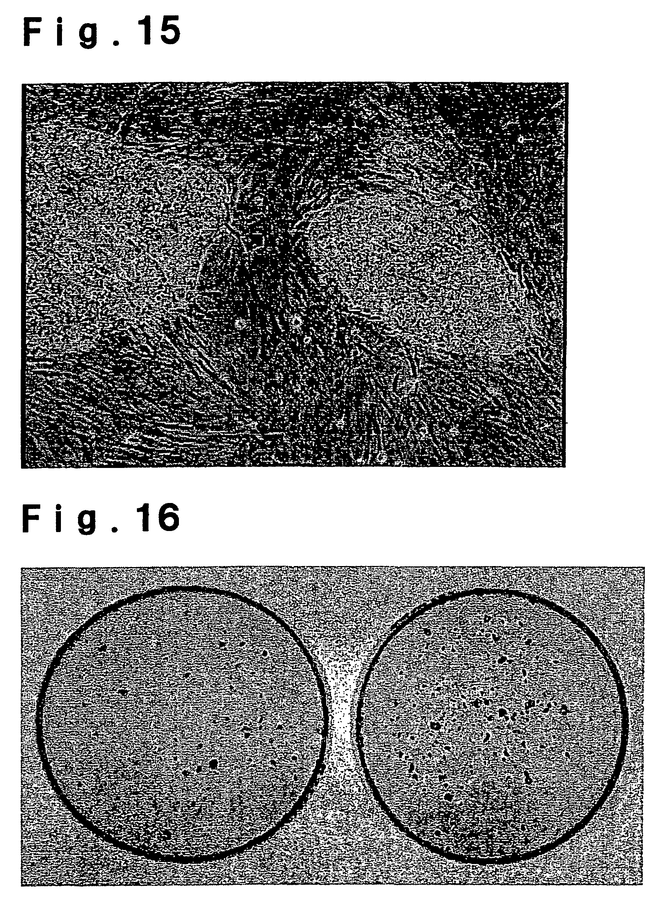

In contrast, though the ES cells in the comparative experiment slightly exceeded the feeder cells in alkaline phosphatase activity after 7 days (Fig. 5), they could not reach the stage at which they were able to be subcultured after 5 days (Figs 12 and 13). After 7 days of prolonged culture, proliferation was recognized to a certain degree, though far below that of the cells in the illustrative experiment (Figs. 14 and 15). The cells in each Petri dish were fixed with paraformaldehyde and ethanol and stained with Vector Red alkaline phosphatase substrate (Vector Labs), and a few successfully stained colonies were observed (Fig. 16).

### EXAMPLE 3

Cynomolgus monkey ES cells subcultured for 35 passages were harvested from three 90-mm Petri dishes and treated by a method similar to that employed in Example 1 to give a cell precipitate. The cell precipitate was resuspended in 1 ml of a cryopreservation medium. consisting of 20% (V/V) [= 22% (W/V)] of DMSO, 40% (V/V) of serum replacement (A), 40% (V/V) of medium (A). The cell suspension was transferred to a serum tube, refrigerated to -80°C at a rate of about 1°C/min in Mr. Frosty (Nalgen) and stored in a liquid nitrogen container for 5 day. The cells were thawed and continuously subcultured in duplicate in 35-mm Petri dishes in the medium described in Suemori et al. (2001 Dev. Dyn. Oct;222(2):273-9).

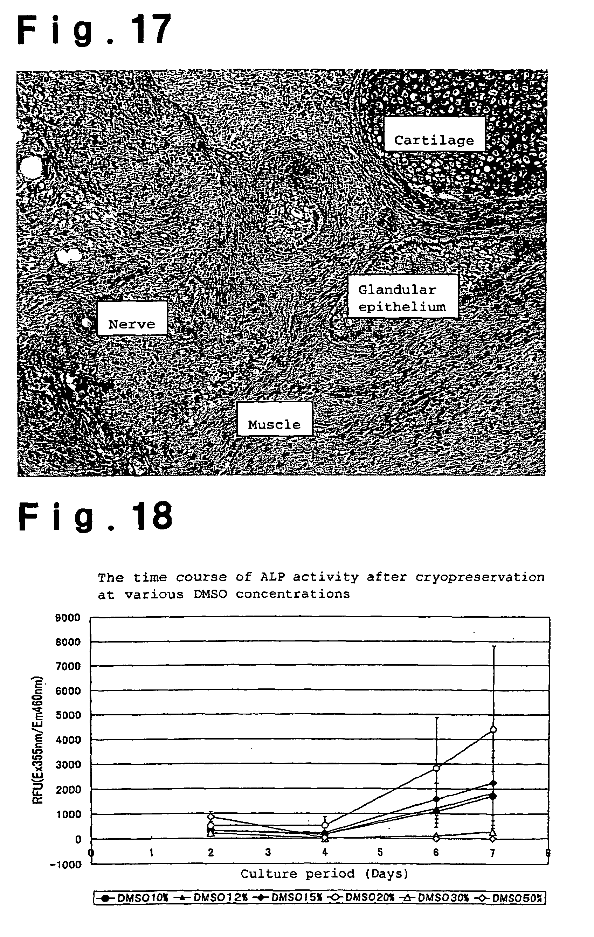

Differentiation potency was assessed after 10 passages. The cells were dissociated from the Petri dishes, and 5×10⁶ cells were suspended in 0.5 ml of a phosphate buffer and intraperitoneally injected into SCID mice. The mice were fed for about 6 weeks, and teratomas were isolated from the abdomen. Observation of the teratomas after Bouin fixation, paraffin sectioning and sematoxylin·eosin staining revealed formation of nerves, muscles and cartilage and demonstrated retention of pluripotency (Fig. 17).

### EXAMPLE 4

Cynomolgus monkey ES cells subcultured for 35 passages were harvested from forty-eight 60-mm Petri dishes each carrying 4×10⁶ cells and treated by a method similar to that employed in Example 1 to give a cell precipitate. The cell precipitate was resuspended in 2 ml of cryopreservation media each consisting of 12% (V/V) [= 13.2 (W/V)], 15% (V/V) [= 16.5% (W/V)], 20% (V/V) [= 22% (W/V)] or 30% (V/V) [= 33% (W/V)] of DMSO and equal concentrations of serum replacement (A) and medium (A) at a cell density of 3.2×10⁷ cells/2 ml, transferred to serum tubes in duplicate, refrigerated to -80°C at a rate of about 1°C/min in Mr. Frosty (Nalgen) and stored in a liquid nitrogen container for 5 days.

In a comparative experiment, cell suspensions in 2 ml of cryopreservation media each consisting of 10% (V/V) [= 11% (W/V)] or 50% (V/V) [= 55% (W/V)] of DMSO and equal concentrations of serum replacement (A) and medium (A) were prepared at a cell density of 3.2×10⁷ cells/2 ml, transferred to serum tubes in duplicate, allowed to stand at 4°C for 2 hours, refrigerated to -80°C at a rate of about 1°C/min in Mr. Frosty (Nalgen) and stored in a liquid nitrogen container for 5 days.

The cells were thawed, plated in 60-mm Petri dishes in duplicate and cultured in the medium described in Suemori et al. (2001 Dev. Dyn. Oct;222(2):273-9).

After 2, 4, 6 and 7 days, ES cell proliferation was assessed by alkaline phosphatase assay as described in Example 1. After 7 days of culture, the cells on each Petri dish was dissociated by tripsinization and counted. The time-dependent increase in alkaline phosphatase activity (Fig. 18) at all the concentrations employed in the illustrative experiment indicates successful. The cell count per Petri dish in the 7 day culture was remarkably greater at DMSO concentrations of 15% (V/V) [= 16.5% (W/V)] and 20% (V/V) [= 22% (W/V)] than that in the comparative experiment, which indicates that practical DMSO concentrations range from 15% (W/V) to 30% (W/v) (Fig. 19). Fig. 16 indicates that DMSO concentrations between 15% (W/V) and 25% (W/V) were preferred.

### EXAMPLE 5

Cynomolgus monkey ES cells subcultured for 32 passages were harvested from thirty-two 60-mm Petri dishes each carrying 2.8×10⁶ cells and treated by a method similar to that employed in Example 1 to give a cell precipitate. The cell precipitate was resuspended in 2 ml of cryopreservation media each consisting of 10% (V/V) [= 12.6% (W/V)], 20% (V/V) [= 25.2% (W/V)] or 30% (V/V) [= 37.8% (W/V)] of glycerin, instead of DMSO, 20% (V/V) of serum replacement (A) and the balance of medium (A) at a cell density of 2.2×10⁷ cells/2 ml, transferred to serum tubes in duplicate, refrigerated to -80°C at a rate of about 1°C/min in Mr. Frosty (Nalgen) and stored in a liquid nitrogen container for 1 day.

In a comparative experiment, cell suspensions in 2 ml of a cryopreservation medium consisting of 5% (V/V) [= 6.3% (W/V)] of glycerin, 20% (V/V) of serum replacement (A) and the balance of medium (A) were prepared at a cell density of 2.2×10⁷ cell/2 ml, transferred to serum tubes in duplicate, allowed to stand at 4°C for 2 hours, refrigerated to -80°C at a rate of about 1°C/min in Mr. Frosty (Nalgen) and stored in a liquid nitrogen container for 1 day.

The cells in each serum tubes were thawed, directly plated on 60-mm Petri dishes in duplicate without centrifugation and cultured in the medium described in Suemori et al. (2001 Dev. Dyn. Oct; 222 (2) :273-9) .

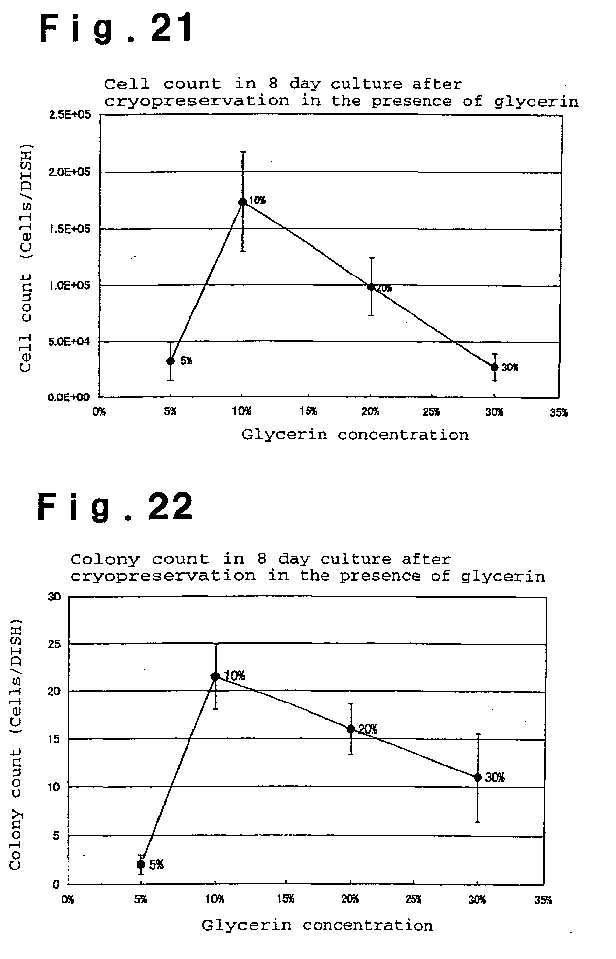

After 2,4,6 and 8 days, ES cell proliferation was assessed by alkaline phosphatase assay as described in Example 1. After 8 days of culture, the cells on each Petri dish was dissociated by tripsinization and counted. Colonies of 1 mm or larger formed on the Petri dishes for the respective concentrations were visually counted. Successful cryopreservation was confirmed by increase in alkaline phosphatase activity after 8 days of culture at all the glycerin concentrations but 5% (V/V) [= 6.3% (W/V)] in the comparative experiment (Fig. 20). The cell count per Petri dish in the 8 day culture was remarkably great at glycerin concentrations of 10% (V/V) [= 12.6% (W/V)] and 20% (V/V) [= 25.2% (W/V)] (Fig. 21), and formation of ES cell colonies was confirmed at glycerin concentrations of 10% (V/V) [= 12.6% (W/V)] or above. These results indicate successful cryopreservation of primate ES cells using a cryoprotectant other than DMSO and preferred cryoprotectant concentrations of from 12% (W/V) to 30% (W/V).

### EXAMPLE 6

Cynomolgus monkey ES cells subcultured for 41 passages were harvested from fifty-six 60-mm Petri dishes each carrying 2.5×10⁶ cells and treated by a method similar to that employed in Example 1 to give a cell precipitate. The cell precipitate was resuspended in 2 ml of a cryopreservation medium consisting of 20% (V/V) [= 22% (W/V)] of DMSO, 40% (V/V) of serum replacement (A) and 40% (V/V) of medium (A) at cell densities of 1.0×10⁷ cells/2 ml, 2.0×10⁷ cells/2 ml and 4.0×10⁷ cells/2 ml, transferred to serum tubes in duplicate, refrigerated to - 80°C at a rate of about 1°C/min in Mr. Frosty (Nalgen) and stored in a liquid nitrogen container for 18 days.

In a comparative experiment, cell suspensions in 2 ml of a cryopreservation medium consisting of 10% (V/V) [= 11% (W/V)], 45% (V/V) of serum replacement (A) and 45% (V/V) of medium (A) were prepared at cell densities of 1.0×10⁷ cells/2 ml, 2.0×10⁷ cells/2 ml and 4.0×10⁷ cells/2 ml, transferred to serum tubes in duplicate, refrigerated to -80°C at a rate of about 1°C/min in Mr. Frosty (Nalgen) and stored in a liquid nitrogen container for 18 days.

The cells in each serum tubes were thawed, plated in 60-mm Petri dishes in duplicate and cultured in the medium described in Suemori et al. (2001 Dev. Dyn. Oct;222(2):273-9).

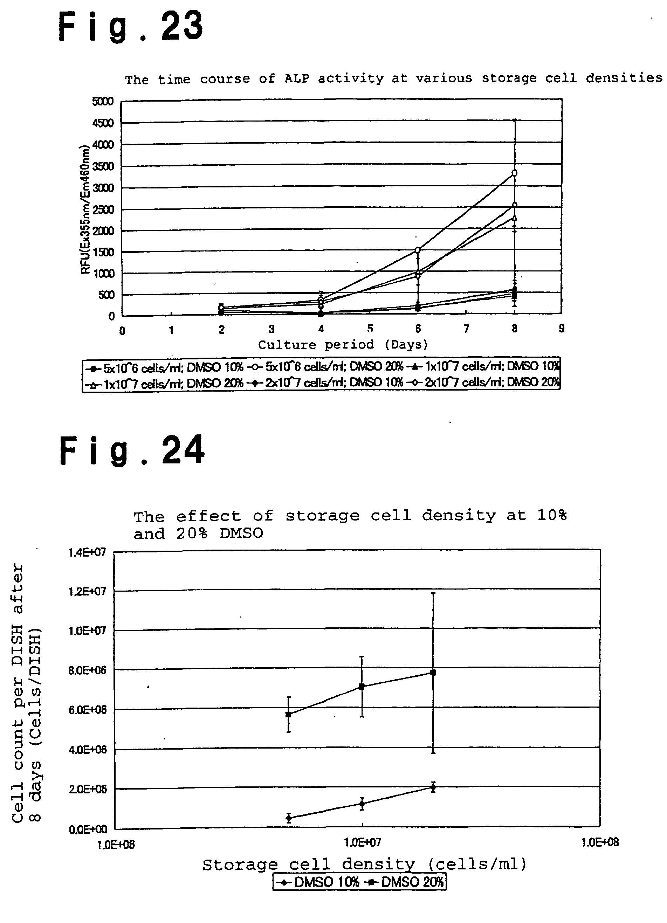

After 2, 4, 6 and 8 days, ES cell proliferation was assessed by alkaline phosphatase assay as described in Example 1. After 8 days of culture, the cells on each Petri dish was dissociated by tripsinization and counted. In the illustrative experiment using the cryopreservation medium containing 20% (V/V) of DMSO, alkaline phosphatase activity increased faster at any cell densities than in the comparative experiment (Fig. 23). The cell count per Petri dish after 8 days of culture was greater than that in the comparative experiment, though tends to increase with the storage cell density, which indicates that increase in cryoprotectant concentration has greater effect than increase in freezing cell density (Fig. 24).

### EXAMPLE 7

Cynomolgus monkey ES cells subcultured for 45 passages were harvested from forty-eight 60-mm Petri dishes each carrying 4.0×10⁶ cells and treated by a method similar to that employed in Example 1 to give a cell precipitate. The cell precipitate was resuspended in 2 ml of cryopreservation media containing 20% (V/V) [= 22% (W/V)] of DMSO with the combination of 10% (V/V) of serum replacement (A) or fetal bovine serum and 70% (V/V) of medium (A), with the combination of 40% (V/V) of serum replacement (A) or fetal bovine serum and 40% (V/V) of medium (A) or with 80% (V/V) of serum replacement (A) or fetal bovine serum at a cell density of 3.2×10⁷ cells/2 ml, transferred to serum tubes in duplicate, refrigerated to -80°C at a rate of about 1°C/min in Mr. Frosty (Nalgen) and stored in a liquid nitrogen container for 5 days. The cells in each serum tubes were thawed, plated in 60-mm Petri dishes in duplicate and cultured in the medium described in Suemori et al. (2001 Dev. Dyn. Oct;222(2):273-9).

After 2, 4, 6 and 7 days, ES cell proliferation was assessed by alkaline phosphatase assay as described in Example 1. After 7 days of culture, the cells on each Petri dish was dissociated by tripsinization and counted. At any concentrations of the medium supplements, alkaline phosphatase activity increased (Fig. 25), and the cell counting per Petri dish after 7 days of culture gave similar results (Fig. 26). Successful cryopreservation was confirmed at any supplement concentrations, though cryopreservation efficiency tended to be better at low supplement concentrations, irrespective of the kind of supplement.

### EXAMPLE 8

Cynomolgus monkey ES cells subcultured for 32 passages were harvested from thirty-two 60-mm Petri dishes each carrying 2.8×10⁶ cells and treated by a method similar to that employed in Example 1 to give a cell precipitate. The cell precipitate was resuspended in 2 ml of a cryopreservation medium consisting of 20% (V/V) [= 22% (W/V)] of DMSO, 20% (V/V) of serum replacement (A) and 60% (V/V) of medium (A) at 2.2×10⁷ cells/2 ml, transferred to serum tubes in duplicate, refrigerated to -80°C at a rate of about 1°C/min in Mr. Frosty (Nalgen) and stored in a liquid nitrogen container for 1 day.

In a comparative experiment, a cell suspension in 2 ml of a cryopreservation medium consisting of 10% (V/V) [= 11% (W/V)] of DMSO, 20% (V/V) of serum replacement (A) and 70% of medium (A) was prepared at a cell density of 2.2×10⁷ cells/2 ml, transferred to serum tubes in duplicate, refrigerated to -80°C at a rate of about 1°C/min in Mr. Frosty (Nalgen) and stored in a liquid nitrogen container for 1 day.

The cells in each serum tubes were thawed, plated in 60-mm Petri dishes in duplicate and cultured in the medium described in Suemori et al. (2001 Dev. Dyn. Oct; 222(2):273-9).

After 2, 4 and 6 days of culture, ES cell proliferation was assessed by alkaline phosphatase assay as described in Example 1. After 6 days of culture, the cells on each Petri dish was dissociated by tripsinization and counted. Alkaline phosphatase activity increased significantly faster in the illustrative experiment employing a DMSO concentration of 20% (V/V) [= 22% (W/V)] than in the comparative experiment (Fig. 27).

The cell count per Petri dish after 6 days of culture at a DMSO concentration of 20% (V/V) [= 22% (W/V)] in the illustrative experiment was 6 times greater than that at a DMSO concentration of 10% (V/V) [= 11% (W/V)] in the comparative experiment (Fig. 28). Though Example 7 indicated that low supplement concentrations facilitate cryopreservation, it is demonstrated that low supplement concentrations do not have as much effect as high cryoprotectant concentrations. It is also confirmed that low supplement concentrations are more effective when combined with high concentrations of DMSO.

### INDUSTRIAL APPLICABILITY

The present invention enables simple cryopreservation of primate ES cells with high viability. Cryopreservation using a serum replacement containing albumin, insulin and transferrin can prevent differentiation induced by unknown factors in serum. Besides, because all the procedures from culture through storage can be carried out without sera, use of ES cells obtained by the present invention in regenerative medicine and gene therapy can obviate risks of contamination by unknown pathogens in sera.

## Claims

1. A cryopreservation medium for primate embryonic stem cells containing a cryoprotectant at a concentration of from 12% (W/V) to 50% (W/V).

2. The cryopreservation medium according to Claim 1, wherein the concentration of the cryoprotectant is from 15% (W/V) to 30% (W/V).

3. The cryopreservation medium according to Claim 1 or 2, which contains at least one component selected from the group consisting of dimethyl sulfoxide, glycerin, ethylene glycol, propylene glycol and polyvinyl pyrrolidone as the cryoprotectant.

4. The cryopreservation medium according to any one of Claims 1 to 3, wherein the cryoprotectant is dimethyl sulfoxide and/or glycerin.

5. The cryopreservation medium according to Claim 4, wherein the cryoprotectant is dimethyl sulfoxide, and its concentration is from 15% (W/V) to 30% (W/V).

6. The cryopreservation medium according to Claim 4, wherein the cryoprotectant is glycerin, and its concentration is from 12% (W/V) to 30% (W/V).

7. The cryopreservation medium according to any one of Claims 1 to 6, which contains, in addition to the cryoprotectant, serum and/or a serum replacement at a concentration of from 10% (W/V) to 85% (W/V).

8. The cryopreservation medium according to any one of Claims 1 to 6, which contains, in addition to the cryoprotectant, serum at a concentration of from 10% (W/V) to 50% (W/V).

9. The cryopreservation medium according to Claim 7 or 8, wherein the serum is fatal bovine serum.

10. The cryopreservation medium according to Claim 7, wherein the serum replacement contains at least one component selected from the group consisting of albumin or albumin substitutes, transferrin or transferrin substitutes, and insulin or insulin substitutes.

11. The cryopreservation medium according to Claim 7 or 10, wherein the serum replacement contains at least one component selected from the group consisting of albumin or albumin substitutes, transferrin or transferrin substitutes, and insulin or insulin substitutes and at least one component selected from the group consisting of amino acids, vitamins, antioxidants, collagen precursors and trace elements.

12. The cryopreservation medium according to any one of Claims 7 to 11, which contains, in addition to the cryoprotectant and serum and/or serum replacement, a basal medium for animal tissue culture containing at least from 0.3% (W/V) to 5% (W/V) of glucose, at a concentration of from 10% (W/V) to 75% (W/V).

13. A cryopreservation method for primate embryonic stem cells using the cryopreservation medium as defined in any one of Claims 1 to 12, which comprises a step of suspending primate embryonic stem cells in the cryopreservation medium, and a refrigeration step of freezing the suspension of the primate embryonic stem cells in the cryopreservation medium by cooling it to -80°C or below, and a preservation step of storing the frozen suspension of primate embryonic stem cells in the cryopreservation medium.

14. The cryopreservation method according to Claim 13, wherein the cooling rate in the refrigeration step of freezing the suspension of the primate embryonic stem cells in the cryopreservation medium by cooling it to -80°C or below is from 0.5°C to 10°C per minute.
